(19) Europäisches Patentamt / European Patent Office / Office européen des brevets

(11) **EP 2 477 542 B1**

(12) **EUROPEAN PATENT SPECIFICATION**

(45) Date of publication and mention
of the grant of the patent:
**13.04.2016  Bulletin 2016/15**

(21) Application number: **10760047.0**

(22) Date of filing: **06.09.2010**

(51) Int Cl.:
**A61B 5/05** $^{(2006.01)}$     **A61M 25/01** $^{(2006.01)}$

(86) International application number:
**PCT/IB2010/053996**

(87) International publication number:
**WO 2011/030276 (17.03.2011 Gazette 2011/11)**

(54) **APPARATUS AND METHOD FOR CONTROLLING THE MOVEMENT AND FOR LOCALIZATION OF A CATHETER**

VORRICHTUNG UND VERFAHREN ZUR STEUERUNG DER BEWEGUNG UND ZUR POSITIONSBESTIMMUNG EINES KATHETERS

APPAREIL ET PROCÉDÉ POUR COMMANDER LE DÉPLACEMENT D'UN CATHÉTER ET POUR LA LOCALISATION DE CELUI-CI

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **14.09.2009  EP 09170212**

(43) Date of publication of application:
**25.07.2012  Bulletin 2012/30**

(73) Proprietors:
- **Koninklijke Philips N.V.**
  **5656 AE Eindhoven (NL)**
  Designated Contracting States:
  **AL AT BE BG CH CY CZ DK EE ES FI FR GB GR HR HU IE IS IT LI LT LU LV MC MK MT NL NO PL PT RO SE SI SK SM TR**
- **Philips Intellectual Property & Standards GmbH**
  **20099 Hamburg (DE)**
  Designated Contracting States:
  **DE**

(72) Inventor: **GLEICH, Bernhard**
**NL-5656 AE Eindhoven (NL)**

(74) Representative: **van Velzen, Maaike Mathilde**
**Philips Intellectual Property & Standards**
**High Tech Campus 5**
**5656 AE Eindhoven (NL)**

(56) References cited:
**WO-A2-2006/067664     DE-A1- 10 151 778**
**US-A1- 2004 006 301     US-A1- 2007 038 064**

- **TANG XIAO-YING ET AL: "Four Cylinder Halbach Array for Magnetic Navigation System", BIOINFORMATICS AND BIOMEDICAL ENGINEERING , 2009. ICBBE 2009. 3RD INTERNATIONAL CONFERENCE ON, IEEE, PISCATAWAY, NJ, USA, 11 June 2009 (2009-06-11), pages 1-4, XP031488656, ISBN: 978-1-4244-2901-1**

## Description

FIELD OF THE INVENTION

The present invention relates to an apparatus for controlling the movement of a catheter through an object and for localizing the catheter within the object, said catheter comprising a magnetic element at or near its tip. Further, the present invention relates to a method for controlling an apparatus according to the present invention to control the movement of a catheter through an object and localize the catheter within the object. Further, the present invention relates to a computer program for implementing said method on a computer and for controlling such an apparatus.

BACKGROUND OF THE INVENTION

Magnetic Particle Imaging (MPI) is an emerging medical imaging modality. The first versions of MPI were two-dimensional in that they produced two-dimensional images. Future versions will be three-dimensional (3D). A time-dependent, or 4D, image of a non-static object can be created by combining a temporal sequence of 3D images to a movie, provided the object does not significantly change during the data acquisition for a single 3D image.

MPI is a reconstructive imaging method, like Computed Tomography (CT) or Magnetic Resonance Imaging (MRI). Accordingly, an MP image of an object's volume of interest is generated in two steps. The first step, referred to as data acquisition, is performed using an MPI scanner. The MPI scanner has means to generate a static magnetic gradient field, called "selection field", which has a single field free point (FFP) at the isocenter of the scanner. In addition, the scanner has means to generate a time-dependent, spatially nearly homogeneous magnetic field. Actually, this field is obtained by superposing a rapidly changing field with a small amplitude, called "drive field", and a slowly varying field with a large amplitude, called "focus field". By adding the time-dependent drive and focus fields to the static selection field, the FFP may be moved along a predetermined FFP trajectory throughout a volume of scanning surrounding the isocenter. The scanner also has an arrangement of one or more, e.g. three, receive coils and can record any voltages induced in these coils. For the data acquisition, the object to be imaged is placed in the scanner such that the object's volume of interest is enclosed by the scanner's field of view, which is a subset of the volume of scanning.

The object must contain magnetic nanoparticles; if the object is an animal or a patient, a contrast agent containing such particles is administered to the animal or patient prior to the scan. During the data acquisition, the MPI scanner steers the FFP along a deliberately chosen trajectory that traces out the volume of scanning, or at least the field of view. The magnetic nanoparticles within the object experience a changing magnetic field and respond by changing their magnetization. The changing magnetization of the nanoparticles induces a time dependent voltage in each of the receive coils. This voltage is sampled in a receiver associated with the receive coil. The samples output by the receivers are recorded and constitute the acquired data. The parameters that control the details of the data acquisition make up the scan protocol.

In the second step of the image generation, referred to as image reconstruction, the image is computed, or reconstructed, from the data acquired in the first step. The image is a discrete 3D array of data that represents a sampled approximation to the position-dependent concentration of the magnetic nanoparticles in the field of view. The reconstruction is generally performed by a computer, which executes a suitable computer program. Computer and computer program realize a reconstruction algorithm. The reconstruction algorithm is based on a mathematical model of the data acquisition. As with all reconstructive imaging methods, this model is an integral operator that acts on the acquired data; the reconstruction algorithm tries to undo, to the extent possible, the action of the model.

Such an MPI apparatus and method have the advantage that they can be used to examine arbitrary examination objects - e. g. human bodies - in a non-destructive manner and without causing any damage and with a high spatial resolution, both close to the surface and remote from the surface of the examination object. Such an arrangement and method are generally known and are first described in DE 101 51 778 A1 and in Gleich, B. and Weizenecker, J. (2005), "Tomographic imaging using the nonlinear response of magnetic particles" in nature, vol. 435, pp. 1214-1217. The arrangement and method for magnetic particle imaging (MPI) described in that publication take advantage of the non-linear magnetization curve of small magnetic particles.

For the movement of a catheter within a patient's body there are many robotic catheter systems. Robotic catheter steering has two advantages. For less trained operators, they may greatly improve speed and accuracy of a catheter procedure. For long procedures, like electrophysiology procedures (EP), they reduce X-ray dosage for the patient. Systems either operate mechanically or by magnetic fields as in the stereotaxis system in which homogeneous magnetic fields bend the catheter.

Such a system is, for instance, known from US 2003/0125752 A1. The movement of a catheter through a medium, which may be living tissue such as a human brain, is controlled in this system by mechanically pushing a flexible catheter having a magnetic tip through the medium and applying a magnetic field having a magnitude and a direction that guides the mechanically-pushed catheter tip stepwise along a desired path. The magnetic field is controlled in the magnetic stereotaxis system by a processor using an adaptation of a PID (proportional, integral, and derivative) feedback method. The magnetic fields are applied by superconducting coils, and the currents applied through the coils are selected to minimize a current metric.

## SUMMARY OF THE INVENTION

It is an object of the present invention to provide an improved apparatus and method for controlling the movement of a catheter through an object, which is also able to localizing the catheter within the object.

In a first aspect of the present invention an apparatus is presented comprising:

- selection means comprising a selection field signal generator unit and selection field elements, in particular selection field magnets or coils, for generating a magnetic selection field having a pattern in space of its magnetic field strength such that a first sub-zone having a low magnetic field strength and a second sub-zone having a higher magnetic field strength are formed in a field of view,
- drive means comprising a drive field signal generator unit and drive field coils for changing the position in space of the two sub-zones in the field of view by means of a magnetic drive field so that the magnetization of magnetic material in the field of view changes locally,
- receiving means comprising at least one signal receiving unit and at least one receiving coil for acquiring detection signals, which detection signals depend on the magnetization in the field of view, which magnetization is influenced by the change in the position in space of the first and second sub-zone,
- control means for controlling said signal generator units to generate and provide control currents to the respective field coils to generate appropriate magnetic fields for moving the catheter through the object in a direction instructed by movement commands and for localizing the catheter within the object, and
- processing means for processing said detection signals acquired when appropriate magnetic fields are applied for localizing the catheter within the object and for determining the position of the magnetic element of the catheter within the object from the processed detection signals.

In a further aspect of the present invention a corresponding method for controlling such an apparatus is presented.

In still a further aspect of the present invention a computer program is presented comprising program code means for causing a computer to control the apparatus according to the present invention to carry out the steps of the method according to the present invention when said computer program is carried out on the computer.

Preferred embodiments of the invention are defined in the dependent claims. It shall be understood that the claimed method and the claimed computer program have similar and/or identical preferred embodiments as the claimed apparatus and as defined in the dependent claims.

It has been recognized by the inventors that a major limitation of the known magnetic stereotaxis systems is the low magnetic field strength of the magnetic fields (e.g. 100 mT), since the contact forces to the heart muscle are considered to be much lower than optimal. One main application of such stereotaxis systems and the invention are electrophysiologic measurements and ablations at the heart. For those applications a catheter (including an electrode) must be pressed against the heart muscle, in particular for ablations. The stronger the magnetic field is, the higher the torque and, thus, force that can be exerted. It has further been recognized that a second drawback of the stereotaxis system is the low speed of magnetic field change.

Hence, it is one idea of the present invention to use parts of a known MPI apparatus and method for generating the required magnetic fields for the catheter steering and, thus, to replace the magnetic stereotaxis system by an MPI system, which is adapted accordingly. In particular, some of the field coils of the known MPI apparatus are used for generating the appropriate magnetic fields, and the control unit of the MPI apparatus is adapted for controlling the respective signal generator units to generate and provide control currents to the respective field coils to generate appropriate magnetic fields by which the catheter is moved through the object. The control unit is also provided with movement commands indicating the direction of movement of the catheter, from which the control unit generates the control commands for the signal generator units.

As the MPI hardware, in particular the various field coils, generally (but not exclusively) enclose the object (patient), the magnetic fields generated by the coils of the MPI system can be substantially larger (e.g. 400 mT) than the magnetic fields produced by the current stereotaxis systems (e.g. 100 mT). Hence, the catheter can be moved much more quickly, with less movement errors and with higher accuracy. Further, an MPI system is much faster, in particular the magnetic fields can be modified much faster than in a stereotaxis system, e.g. by two orders of magnitude. In addition, higher torques can be exerted so that higher speeds against friction can be achieved. The higher rate of the magnetic field changes can be realized particularly since the field generator can be brought more closely to the patient (e.g. since not space for a voluminous x-ray system is needed) and since the MPI system requires large current sources for providing the required currents in an MPI data acquisition (e.g. for localization and imaging), which are thus available in the system anyhow and which can thus be advantageously exploited for the desired catheter movement.

Still further, the use of the principles and of the hardware of an MPI system allows to additionally localize the catheter within the object. The movement and the localization of the catheter can thus be done with the apparatus according to the present invention alternately and almost simultaneously without additional equipment, such as additional hardware for localization, e.g. a camera system or an x-ray system for detecting markers applied to the catheter as conventionally used. For the localization the known MPI principles of imaging magnetic particles an object, as for instance described in the above mentioned documents, are applied, i.e. the control unit then generates control commands for the signal generator units to generate and provide control currents to the respective field coils to generate appropriate magnetic fields for imaging the catheter, in particular the magnetic element at or near its tip. For this purpose, the magnetic element is made from or contains magnetic material that is appropriate for this purpose, e.g. ferromagnetic material, such as Resovist. The applied selection field then has a pattern in space of its magnetic field strength such that a first sub-zone, i.e. the generally called field-free-point (FFP), having a low magnetic field strength where the magnetization of the magnetic particles is not saturated and a second sub-zone having a higher magnetic field strength where the magnetization of the magnetic particles is saturated, and the field-free-point is then moved along a predetermined trajectory by the application of appropriate drive magnetic fields and/or (optional) focus magnetic fields (if available).

This enables the apparatus and method according to the present invention to easily check the correct movement and position of the catheter during the intervention without the use of another imaging modality, such as X-ray or CT, and thus reduces the dosage for the patient. Further, no additional hardware is required for this functionality, as is required with the known stereotaxis system.

According to a preferred embodiment said control means is adapted for controlling said signal generator units to generate and provide control currents to the respective field coils to alternately generate appropriate magnetic fields for moving the catheter through the object in a direction instructed by movement commands and for localizing the catheter within the object. Hence, during the movement of the catheter the actual catheter position can be determined and checked at desired time intervals. In this way, the position deviates from the desired position, an immediate correction can be made, either automatically by the apparatus or manually by the user.

According to another embodiment the control means is adapted for converting manual or predetermined movement commands into control signals for controlling said signal generator units. Preferably, an interface for inputting such movement commands to the control unit is provided. Such an interface can be a user interface, such as a keyboard, pointer, computer mouse or joystick, or an interface to another apparatus, such as a navigation unit or navigation tool on a computer, on which, for instance, the movement of the catheter has been planned, e.g. by use of image data of the patient obtained by use of another imaging modality, such as MR or CT. The control unit is then provided with movement commands and "translates" them into control signals for the respective signal generator units so that the appropriate magnetic fields will be generated.

While the catheter can generally be moved within the object solely the forces applied by the magnetic fields, it is preferred in an embodiment to provide, in addition to the movement by the magnetic fields, a forward and backward movement of the catheter by use of a catheter movement means. This supports the movement of the catheter into and out of the object or even solely provides the forces for forward and backward movement, so that the magnetic fields mainly or only have the task to control the direction of movement within the object.

Such catheter movement means for pushing a flexible catheter through a medium are generally known and also used in the described stereotaxis systems. Such a catheter movement means is, for instance, described in US 2003/0125752 A1. But generally, any kind of such catheter movement means can be used here, and the invention is not limited to the embodiment described in this document.

The control means is preferably adapted for controlling said catheter movement means. This enables a controlled coordination of the movement, positioning and localization of the catheter in the object.

Alternatively, the forward and backward movement of the catheter can also be provided manually by the user, and the magnetic fields are only provided for controlling the direction of movement of the catheter, in particular the catheter tip, within the object.

The control means is further preferably adapted for controlling said catheter movement means such that during localization of said catheter no forward or backward movement is applied on the catheter, in particular such that said catheter is kept in position. If no such catheter movement means are provided, the control means controls the signal generator units such that the catheter is not moved during the localization, i.e. the catheter steering fields is switched off or switched to a gradient field, and an MPI sequence is applied for localization. If movement of the catheter is performed manually, the user stops forward (or backward) movement of the catheter during localization. This ensures a higher accuracy of the localization.

Preferably, magnetic focus field coils (and/or eventually the magnetic drive field coils) of the apparatus are used for the movement of the catheter through the object. These coils are able to generate sufficiently homogenous fields in various directions at a sufficiently high speed and with sufficiently large field strength, that are required for the catheter movement. The use of these coils provides thus also a much higher flexibility than the known stereotaxis systems, since generally the magnetic fields can be generated in any desired direction.

By use of the homogenous magnetic fields a torque can be exerted on an suitable magnetic object, e.g. the magnetic element at or close to the catheter tip. The torque is at least sufficient for pressing the magnetic element and, thus, the catheter tip to the side, e.g. to force the catheter to follow a certain direction or to follow one of a number of available paths, or to press the catheter against something, e.g. the heart muscle. Preferably, an additional force is required from outside for forward/backward movement, as mentioned above, but it is also possible to apply a strong gradient field to exert a (relatively small) force in one direction for forward or backward movement of the catheter.

Still further, in an embodiment the control means is adapted for controlling said selection field signal generator unit to generate and provide no control current to the selection field coils while magnetic fields are generated by said focus field coils and/or said drive field coils for moving the catheter through the object in a direction instructed by movement commands. This avoids any disturbances of the catheter positioning (particularly caused by magnetic fields generated by the selection field coils) during the movement by use of the focus and/or drive field coils.

BRIEF DESCRIPTION OF THE DRAWINGS

These and other aspects of the invention will be apparent from and elucidated with reference to the embodiment(s) described hereinafter. In the following drawings

Fig. 1 shows a first embodiment of an MPI apparatus,
Fig. 2 shows an example of the selection field pattern produced by an apparatus as shown in Fig. 1,
Fig. 3 shows a second embodiment of an MPI apparatus,
Fig. 4 shows a block diagram of an MPI apparatus according to the present invention, and
Fig. 5 shows a diagram illustrating the method according to the present invention.

DETAILED DESCRIPTION OF THE INVENTION

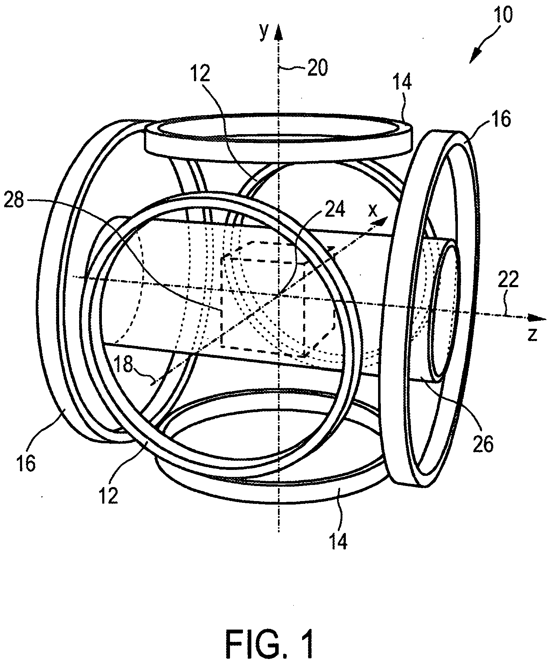

Before the details of the present invention shall be explained, basics of magnetic particle imaging shall be explained in detail with reference to Figs. 1 to 4. In particular, two embodiments of an MPI scanner for medical diagnostics will be described. An informal description of the data acquisition is also given. The similarities and differences between the two embodiments will be pointed out.

The first embodiment 10 of an MPI scanner shown in Fig. 1 has three prominent pairs 12, 14, 16 of coaxial parallel circular coils, each pair being arranged as illustrated in Fig. 1. These coil pairs 12, 14, 16 serve to generate the selection field as well as the drive and focus fields. The axes 18, 20, 22 of the three coil pairs 12, 14, 16 are mutually orthogonal and meet in a single point, designated the isocenter 24 of the MPI scanner 10. In addition, these axes 18, 20, 22 serve as the axes of a 3D Cartesian x-y-z coordinate system attached to the isocenter 24. The vertical axis 20 is nominated the y-axis, so that the x and z-axes are horizontal. The coil pairs 12, 14, 16 are also named after their axes. For example, the y-coil pair 14 is formed by the coils at the top and the bottom of the scanner. Moreover, the coil with the positive (negative) y-coordinate is called the $y^+$-($y^-$-coil), and similarly for the remaining coils.

The scanner 10 can be set to direct a predetermined, time dependent electric current through each of these coils 12, 14, 16, and in either direction. If the current flows clockwise around a coil when seen along this coil's axis, it will be taken as positive, otherwise as negative. To generate the static selection field, a constant positive current $I^S$ is made to flow through the $z^+$-coil, and the current $- I^S$ is made to flow through the $z^-$-coil. The z-coil pair 16 then acts as an anti-parallel circular coil pair.

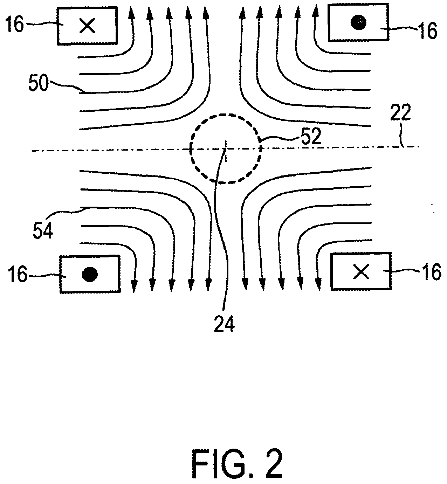

The magnetic selection field which is generally a gradient magnetic field is represented in Fig. 2 by the field lines 50. It has a substantially constant gradient in the direction of the (e.g. horizontal) z-axis 22 of the z-coil pair 16 generating the selection field and reaches the value zero in the isocenter 24 on this axis 22. Starting from this field-free point (not individually shown in Fig. 2), the field strength of the magnetic selection field 50 increases in all three spatial directions as the distance increases from the field-free point. In a first sub-zone or region 52 which is denoted by a dashed line around the isocenter 24 the field strength is so small that the magnetization of particles present in that first sub-zone 52 is not saturated, whereas the magnetization of particles present in a second sub-zone 54 (outside the region 52) is in a state of saturation. The field-free point or first sub-zone 52 of the scanner's field of view 28 is preferably a spatially coherent area; it may also be a punctiform area, a line or a flat area. In the second sub-zone 54 (i.e. in the residual part of the scanner's field of view 28 outside of the first sub-zone 52) the magnetic field strength of the selection field is sufficiently strong to keep the magnetic particles in a state of saturation.

By changing the position of the two sub-zones 52, 54 within the field of view 28, the (overall) magnetization in the field of view 28 changes. By measuring the magnetization in the field of view 28 or physical parameters influenced by the magnetization, information about the spatial distribution of the magnetic particles in the field of view 28 can be obtained. In order to change the relative spatial position of the two sub-zones 52, 54 in the field of view 28, further magnetic fields, i.e. the magnetic drive field, and, if applicable, the magnetic focus field, are superposed to the selection field 50 in the field of view 28 or at least in a part of the field of view 28.

To generate the drive field, a time dependent current $I^D_1$ is made to flow through both x-coils 12, a time dependent current $I^D_2$ through both y-coils 14, and a time dependent current $I^D_3$ through both z-coils 16. Thus, each of the three coil pairs acts as a parallel circular coil pair. Similarly, to generate the focus field, a time dependent current $I^F_1$ is made to flow through both x-coils 12, a current $I^F_2$ through both y-coils 14, and a current $I^F_3$ through both z-coils 16.

It should be noted that the z-coil pair 16 is special: It generates not only its share of the drive and focus fields, but also the selection field. The current flowing through the $z^\pm$-coil is $I^D_3 + I^F_3 + I^S$. The current flowing through the remaining two coil pairs 12, 14 is $I^D_k + I^F_k$, k = 1, 2. Because of their geometry and symmetry, the three coil pairs 12, 14, 16 are well decoupled. This is wanted.

Being generated by an anti-parallel circular coil pair, the selection field is rotationally symmetric about the z-axis, and its z-component is nearly linear in z and independent of x and y in a sizeable volume around the isocenter 24. In particular, the selection field has a single field free point (FFP) at the isocenter. In contrast, the contributions to the drive and focus fields, which are generated by parallel circular coil pairs, are spatially nearly homogeneous in a sizeable volume around the isocenter 24 and parallel to the axis of the respective coil pair. The drive and focus fields jointly generated by all three parallel circular coil pairs are spatially nearly homogeneous and can be given any direction and strength, up to some maximum strength. The drive and focus fields are also time dependent. The difference between the focus field and the drive field is that the focus field varies slowly in time and has a large amplitude while the drive field varies rapidly and has a small amplitude. There are physical and biomedical reasons to treat these fields differently. A rapidly varying field with a large amplitude would be difficult to generate and hazardous to the patient.

The embodiment 10 of the MPI scanner has at least one further pair, preferably three further pairs, of parallel circular coils, again oriented along the x-, y-, and z-axes. These coil pairs, which are not shown in Fig. 1, serve as receive coils. As with the coil pairs 12, 14, 16 for the drive and focus fields, the magnetic field generated by a constant current flowing through one of these receive coil pairs is spatially nearly homogeneous within the field of view and parallel to the axis of the respective coil pair. The receive coils are supposed to be well decoupled. The time dependent voltage induced in a receive coil is amplified and sampled by a receiver attached to this coil. More precisely, to cope with the enormous dynamic range of this signal, the receiver samples the difference between the received signal and a reference signal. The transfer function of the receiver is non-zero from DC up to the point where the expected signal level drops below the noise level.

The embodiment 10 of the MPI scanner shown in Fig. 1 has a cylindrical bore 26 along the z-axis 22, i.e. along the axis of the selection field. All coils are placed outside this bore 26. For the data acquisition, the patient (or object) to be imaged (or treated) is placed in the bore 26 such that the patient's volume of interest - that volume of the patient (or object) that shall be imaged (or treated) - is enclosed by the scanner's field of view 28 - that volume of the scanner whose contents the scanner can image. The patient (or object) is, for instance, placed on a patient table. The field of view 28 is a geometrically simple, isocentric volume in the interior of the bore 26, such as a cube, a ball, or a cylinder. A cubical field of view 28 is illustrated in Fig. 1.

The size of the first sub-zone 52 is dependent on the one hand on the strength of the gradient of the magnetic selection field and on the other hand on the field strength of the magnetic field required for saturation. For a sufficient saturation of the magnetic particles at a magnetic field strength of 80 A/m and a gradient (in a given space direction) of the field strength of the magnetic selection field amounting to $50 \times 10^3$ A/m$^2$, the first sub-zone 52 in which the magnetization of the particles is not saturated has dimensions of about 1 mm (in the given space direction).

The patient's volume of interest is supposed to contain magnetic nanoparticles. Especially prior to a therapeutic and/or diagnostic treatment of, for example, a tumor, the magnetic particles are positioned in the volume of interest, e.g. by means of a liquid comprising the magnetic particles which is injected into the body of the patient (object) or otherwise administered, e.g. orally, to the patient.

An embodiment of magnetic particles comprises, for example, a spherical substrate, for example, of glass which is provided with a soft-magnetic layer which has a thickness of, for example, 5 nm and consists, for example, of an iron-nickel alloy (for example, Permalloy). This layer may be covered, for example, by means of a coating layer which protects the particle against chemically and/or physically aggressive environments, e.g. acids. The magnetic field strength of the magnetic selection field 50 required for the saturation of the magnetization of such particles is dependent on various parameters, e.g. the diameter of the particles, the used magnetic material for the magnetic layer and other parameters.

In the case of e.g. a diameter of 10 $\mu$m, a magnetic field of approximately 800 A/m (corresponding approximately to a flux density of 1 mT) is then required, whereas in the case of a diameter of 100 $\mu$m a magnetic field of 80 A/m suffices. Even smaller values are obtained when a coating of a material having a lower saturation magnetization is chosen or when the thickness of the layer is reduced. Magnetic particles that can generally be used are available on the market under the trade name Resovist.

For further details of the generally usable magnetic particles and particle compositions, the corresponding parts of EP 1304542, WO 2004/091386, WO 2004/091390, WO 2004/091394, WO 2004/091395, WO 2004/091396, WO 2004/091397, WO 2004/091398, WO 2004/091408 are herewith referred to.

In these documents more details of the MPI method in general can be found as well.

The data acquisition starts at time $t_s$ and ends at time $t_e$. During the data acquisition, the x-, y-, and z-coil pairs 12, 14, 16 generate a position- and time dependent magnetic field, the applied field. This is achieved by directing suitable currents through the coils. In effect, the drive and focus fields push the selection field around such that the FFP moves along a preselected FFP trajectory that traces out the volume of scanning - a superset of the field of view. The applied field orientates the magnetic nanoparticles in the patient. As the applied field changes, the resulting magnetization changes too, though it responds nonlinearly to the applied field. The sum of the changing applied field and the changing magnetization induces a time dependent voltage $V_k$ across the terminals of receive coil pair along the $x_k$-axis. The associated receiver converts this voltage to a signal $S_k(t)$, which it samples and outputs.

It is advantageous to receive or to detect signals from the magnetic particles located in the first sub-zone 52 in another frequency band (shifted to higher frequencies) than the frequency band of the magnetic drive field variations. This is possible because frequency components of higher harmonics of the magnetic drive field frequency occur due to a change in magnetization of the magnetic particles in the scanner's field of view 28 as a result of the non-linearity of the magnetization characteristics.

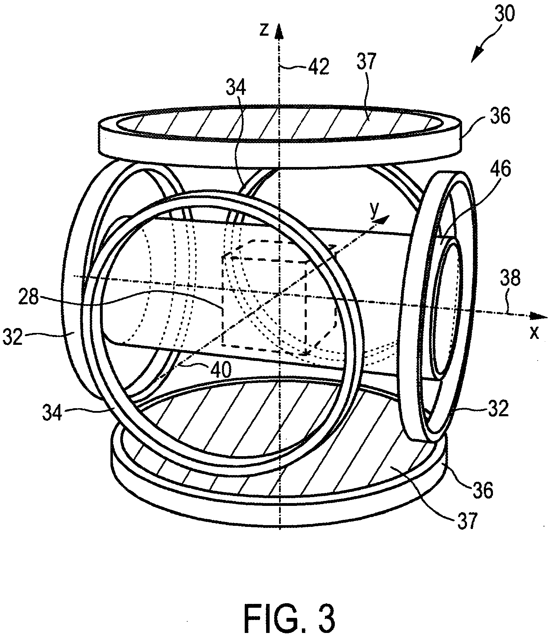

Like the first embodiment 10 shown in Fig. 1, the second embodiment 30 of the MPI scanner shown in Fig. 3 has three circular and mutually orthogonal coil pairs 32, 34, 36, but these coil pairs 32, 34, 36 generate the selection field and the focus field only. The z-coils 36, which again generate the selection field, are filled with ferromagnetic material 37. The z-axis 42 of this embodiment 30 is oriented vertically, while the x- and y-axes 38, 40 are oriented horizontally. The bore 46 of the scanner is parallel to the x-axis 38 and, thus, perpendicular to the axis 42 of the selection field. The drive field is generated by a solenoid (not shown) along the x-axis 38 and by pairs of saddle coils (not shown) along the two remaining axes 40, 42. These coils are wound around a tube which forms the bore. The drive field coils also serve as receive coils. The signals picked up by the receive coils are sent through a high-pass filter that suppresses the contribution caused by the applied field.

To give a few typical parameters of such an embodiment: The z-gradient of the selection field, G, has a strength of $G/\mu_0 = 2.5$ T/m, where $\mu_0$ is the vacuum permeability. The selection field generated does either not vary at all over the time or the variation is comparably slow, preferably between approximately 1 Hz and approximately 100 Hz. The temporal frequency spectrum of the drive field is concentrated in a narrow band around 25 kHz (up to approximately 100 kHz). The useful frequency spectrum of the received signals lies between 50 kHz and 1 MHz (eventually up to approximately 10 MHz).

The bore has a diameter of 120 mm. The biggest cube 48 that fits into the bore 46 has an edge length of

$$120 \ \mathrm{mm}/\sqrt{2} \approx 84 \ \mathrm{mm}.$$

As shown in the above embodiments the various magnetic fields can be generated by coils of the same coils pairs and by providing these coils with appropriately generated currents. However, and especially for the purpose of a signal interpretation with a higher signal to noise ratio, it may be advantageous when the temporally constant (or quasi constant) selection field and the temporally variable drive field and focus field are generated by separate coil pairs. Generally, coil pairs of the Helmholtz type can be used for these coils, which are generally known, e.g. from the field of magnetic resonance apparatus with open magnets (open MRI) in which a radio frequency (RF) coil pair is situated above and below the region of interest, said RF coil pair being capable of generating a temporally variable magnetic field. Therefore, the construction of such coils need not be further elaborated herein.

In an alternative embodiment for the generation of the selection field, permanent magnets (not shown) can be used. In the space between two poles of such (opposing) permanent magnets (not shown) there is formed a magnetic field which is similar to that shown in Fig. 2, that is, when the opposing poles have the same polarity. In another alternative embodiment, the selection field can be generated by a mixture of at least one permanent magnet and at least one coil.

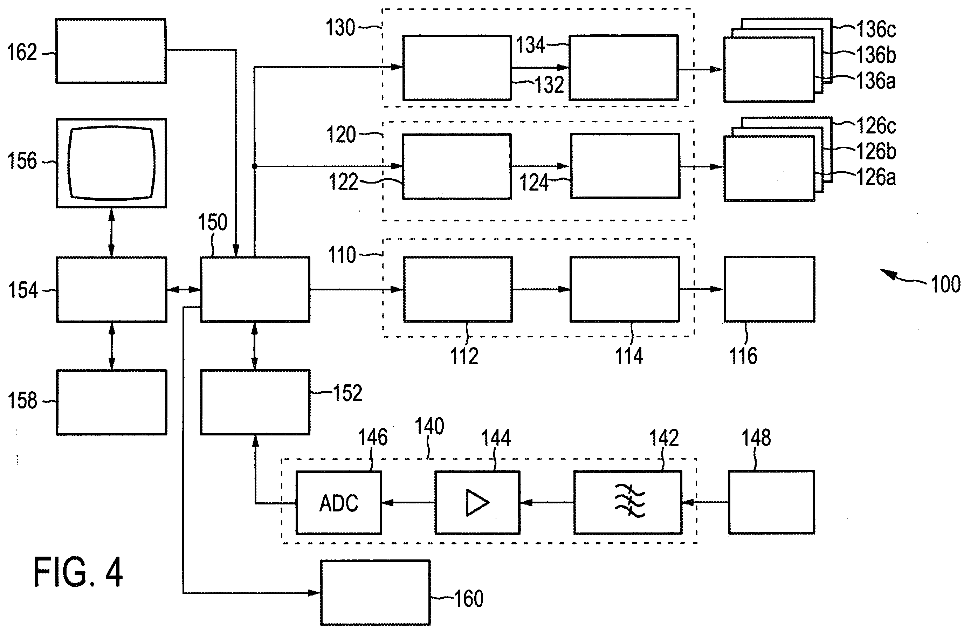

Fig. 4 shows a general block diagram of an MPI apparatus 10 according to the present invention. The general principles of magnetic particle imaging and of magnetic resonance imaging explained above are valid and applicable to this embodiment as well, unless otherwise specified.

The embodiment of the apparatus 100 shown in Fig. 4 comprises a set of various coils for generating the desired magnetic fields. First, the coils and their functions in a MPI mode shall be explained.

For generating the magnetic (gradient) selection field explained above, selection means are provided comprising a set of selection field (SF) coils 116, preferably comprising at least one pair of coil elements. The selection means further comprises a selection field signal generator unit 110. Preferably, a separate generator subunit is provided for each coil element (or each pair of coil elements) of the set 116 of selection field coils. Said selection field signal generator unit 110 comprises a controllable selection field current source 112 (generally including an amplifier) and a filter unit 114 which provide the respective section field coil element with the selection field current to individually set the gradient strength of the selection field in the desired direction. Preferably, a DC current is provided. If the selection field coil elements are arranged as opposed coils, e.g. on opposite sides of the field of view, the selection field currents of opposed coils are preferably oppositely oriented.

The selection field signal generator unit 110 is controlled by a control unit 150, which preferably controls the selection field current generation 110 such that the sum of the field strength and the sum of the gradient strength of all spatial fractions of the selection field is maintained at a predefined level.

For generation of a magnetic focus field the apparatus 100 further comprises focus means comprising a set of focus field (FF) coils, preferably comprising three pairs 126a, 126b, 126c of oppositely arranged focus field coil elements. Said magnetic focus field is generally used for changing the position in space of the region of action. The focus field coils are controlled by a focus field signal generator unit 120, preferably comprising a separate focus field signal generation subunit for each coil element (or at least each pair of coil elements) of said set of focus field coils. Said focus field signal generator unit 120 comprises a focus field current source 122 (preferably comprising a current amplifier) and a filter unit 124 for providing a focus field current to the respective coil of said subset of coils 126a, 126b, 126c which shall be used for generating the magnetic focus field. The focus field current unit 120 is also controlled by the control unit 150.

For generation of the magnetic drive field the apparatus 100 further comprises drive means comprising a subset of drive field (DF) coils, preferably comprising three pairs 136a, 136b, 136c of oppositely arranged drive field coil elements. The drive field coils are controlled by a drive field signal generator unit 130, preferably comprising a separate drive field signal generation subunit for each coil element (or at least each pair of coil elements) of said set of drive field coils. Said drive field signal generator unit 130 comprises a drive field current source 41 (preferably including a current amplifier) and a filter unit 42 for providing a drive field current to the respective drive field coil. The drive field current source 41 is adapted for generating an AC current and is also controlled by the control unit 150.

For signal detection receiving means 148, in particular a receiving coil, and a signal receiving unit 140, which receives signals detected by said receiving means 148, are provided. Said signal receiving unit 140 comprises a filter unit 142 for filtering the received detection signals. The aim of this filtering is to separate measured values, which are caused by the magnetization in the examination area which is influenced by the change in position of the two part-regions (52, 54), from other, interfering signals. To this end, the filter unit 142 may be designed for example such that signals which have temporal frequencies that are smaller than the temporal frequencies with which the receiving coil 148 is operated, or smaller than twice these temporal frequencies, do not pass the filter unit 142. The signals are then transmitted via an amplifier unit 144 to an analog/digital converter 146 (ADC). The digitalized signals produced by the analog/digital converter 146 are fed to an image processing unit (also called reconstruction means) 152, which reconstructs the spatial distribution of the magnetic particles from these signals and the respective position which the first part-region 52 of the first magnetic field in the examination area assumed during receipt of the respective signal and which the image processing unit 152 obtains from the control unit 150. The reconstructed spatial distribution of the magnetic particles is finally transmitted via the control means 150 to a computer 154, which displays it on a monitor 156. Thus, an image can be displayed showing the distribution of magnetic particles in the field of view of the examination area.

Further, an input unit 158 is provided, for example a keyboard. A user is therefore able to set the desired direction of the highest resolution and in turn receives the respective image of the region of action on the monitor 156. If the critical direction, in which the highest resolution is needed, deviates from the direction set first by the user, the user can still vary the direction manually in order to produce a further image with an improved imaging resolution. This resolution improvement process can also be operated automatically by the control unit 150 and the computer 154. The control unit 150 in this embodiment sets the gradient field in a first direction which is automatically estimated or set as start value by the user. The direction of the gradient field is then varied stepwise until the resolution of the thereby received images, which are compared by the computer 154, is maximal, respectively not improved anymore. The most critical direction can therefore be found respectively adapted automatically in order to receive the highest possible resolution.

According to the present invention the control unit 150 is adapted for controlling the signal generator units 110, 120, 130, in particular the focus field signal generator unit 120 and/or the drive field signal generator unit 130, to generate and provide control currents to the respective field coils, in particular the focus field coils 126a, 126b, 126c and/or the drive field coils 136a, 136b, 136c, to generate appropriate magnetic fields for moving a catheter through the object, in particular the patient, in a direction instructed by movement commands. Preferably, the focus field coils 126a, 126b, 126c are used for this purpose.

Preferably, by use of homogenous magnetic fields, e.g. generated by said focus field coils 126a, 126b, 126c, a torque can be exerted on an suitable magnetic object, e.g. a magnetic element at or close to the catheter tip. The torque is at least sufficient for pressing the magnetic element and, thus, the catheter tip to the side, e.g. to force the catheter to follow a certain direction or to follow one of a number of available paths, or to press the catheter against something, e.g. the heart muscle. Preferably, an additional force is required from outside for forward/backward movement, as mentioned above, but it is also possible to apply a strong gradient field to exert a (relatively small) force in one direction for forward or backward movement of the catheter, as will be explained below.

For inputting movement commands, an interface 162 is provided. Said interface 162 can be implemented in various ways. For instance, said interface 162 can be a user interface by which the user can manually input user commands, such as via a keyboard, a console, a joystick or a navigation tool, e.g. installed on a separate computer (not shown). In another implementation said interface 162 is an interface for connection to an external device for movement control, such as a navigation unit, by use of which the movement of the catheter for the current intervention has been planned in advance, e.g. based on image data of the object acquired in advance by another imaging modality, such as MR (Magnetic Resonance) or CT (Computed Tomography), or by use of image data acquired by use of the same MPI apparatus. The interface 162 then receives information about the desired movement of the catheter within the object, and either the interface 162 or the control unit 150 is able to "translate" said commands into movement commands for the respective signal generator units.

Hence, in effect, the apparatus according to the present invention is able to move the catheter to the object, in particular to control the direction of movement of the catheter, based on movement commands, irrespective in which form and by whom or what the movement commands have been provided.

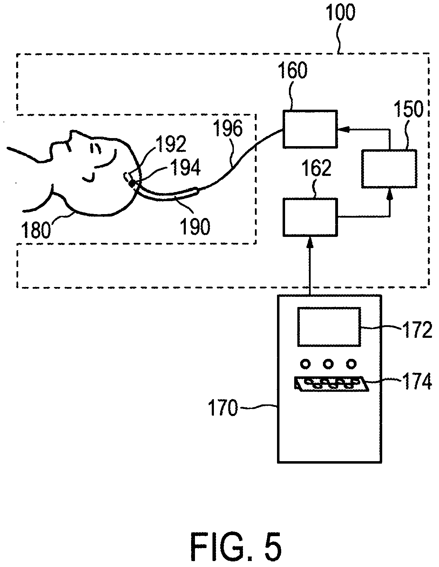

In addition, by use of the apparatus according to the present invention it is easily possible to localize the catheter 190 within the object 180 during the intervention (see Fig. 5 illustrating the method of the invention in a simple diagram). Since the catheter 190 is provided with a magnetic element 194 at or near its tip 192, by use of the known principles of the MPI method and apparatus the location of the magnetic element 194, and thus, of the catheter 190 within the object 180 (here a patient's head) can be determined.

For instance, by use of the known MPI method the position can be retrieved from the acquired detection signals after application of magnetic fields according to the MPI scheme for determining the location of the magnetic element. A position information can be generated or the current position of the magnetic element can be indicated in a predetermined image of the object 180, which may have been previously reconstructed based on data acquired by use of another imaging modality or the same MPI apparatus. Of course, if image data obtained by another imaging modality are used for this purpose, a registration step is generally required for registering these image data to the current detection signals (or image data reconstructed therefrom), for which purpose known registration algorithms can be used.

For instance, for moving the catheter through the object, the focus field coils are preferably used, by which a homogenous magnetic field is generated in the desired direction to effect the desired movement of the catheter. For localization, however, the homogenous steering field (i.e. the focus field) is no longer applied in the same way, but is generally switched to a gradient field (applied by the selection field coil 116 (which can also be a selection field magnet or a number of selection field coils)), and the magnetic fields are applied for moving the field free point along a trajectory through the field of view. In this way the magnetic element attached to the catheter can be detected. During this MPI sequence, that is applied for localization, the forces on the catheter vanish. For faster switching, between modes, some gradient component can persist during catheter steering.

The forward and backward movement of the catheter can be performed manually so that the magnetic fields only (or mainly) are responsible for controlling the direction of movement of the catheter tip. However, it is also possible that the magnetic fields are strong enough to (alone) apply also the forces the forward (and, if needed, backward) movement of the catheter or at least support the forward (or backward) movement. In still a further embodiment a catheter movement unit 160, such as an advancement mechanism comprising a motor as shown in US 2003/0125752 A1, can be provided by which said forward and backward movement is effected. In this case, the catheter 190 is preferably connected to the catheter movement unit 160 by a push wire 196. Generally, any kind of device that can provide a forward (and eventually, backward) movement of a catheter can be used here for this purpose.

The catheter movement device 160 can be controlled directly by the user. Preferably, however, it is controlled by the control unit 150 which also enables to stop the movement of the catheter easily, when localization of the catheter is done.

Fig. 5 illustrates the method according to the present invention in a simple example. Only a few elements of the apparatus 100 according to the present invention are shown.

As can be seen from Fig. 5 the catheter 190 is introduced into the patient's head 180. In particular, the tip 192 of the catheter 190 is inserted, at which tip 192 a magnetic element 194 comprising (or consisting of) easily magnetizable material, e.g. a soft magnetic foil. In particular, a magnetic material is used which enables movement by the application of magnetic fields and localization (imaging) by the known MPI principle and hardware.

The push wire of the catheter 190 is connected to the catheter movement device 160 for forward and backward movement of the catheter under control of the control unit 150. Via the interface 162 movement commands are received from an external movement control unit 170 comprising a display 172, e.g. for displaying preacquired image data of the patient's head, and an operator control 174 for inserting control commands for planning the movement of the catheter.

In a practical intervention the surgeon will plan the intervention using the movement control unit 170. The navigation plan, in particular the movement control commands, are then provided via the interface 162 to the control unit 150 of the MPI apparatus 100. The control unit 150 then controls the catheter movement device 160 as well as the coils (not shown) to provide the movement of the catheter 190 within the patient's head. At desired (e.g. regular) intervals the movement of the catheter 190 is stopped and its current position is acquired by applying an MPI sequence, preferably by moving the FFP along a trajectory through the area in which the magnetic element 194 might be currently located, and acquiring detection signals, which are then processed to get the current position of the magnetic element 194.

Thus, a direct feedback can be obtained whether or not the actual position of the catheter tip 192 corresponds to the desired position, so that immediate corrections can be made, either manually or by the control unit 150. For this purpose, preferably, the obtained position data from the localization are fed back to the control unit 150 and/or a feedback is given to the user, for instance by issuing a warning via the display 156 of the apparatus 100 and/or the display 172 of the catheter movement unit 170, so that the user can take immediate action for correction of the current position.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measured cannot be used to advantage.

Any reference signs in the claims should not be construed as limiting the scope.

## Claims

1. An apparatus (100) for controlling the movement of a catheter (190) through an object (180) and for localizing the catheter (190) within the object (180), said catheter (190) comprising a magnetic element (194) at or near its tip (192), which apparatus comprises:

   - selection means comprising a selection field signal generator unit (110) and selection field elements (116), in particular selection field magnets or coils, for generating a magnetic selection field (50) having a pattern in space of its magnetic field strength such that a first sub-zone (52) having a low magnetic field strength and a second sub-zone (54) having a higher magnetic field strength are formed in a field of view (28),
   - drive means comprising a drive field signal generator unit (130) and drive field coils (136a, 136b, 136c) for changing the position in space of the two sub-zones (52, 54) in the field of view (28) by means of a magnetic drive field so that the magnetization of magnetic material in the field of view changes locally,
   - receiving means comprising at least one signal receiving unit (140) and at least one receiving coil (148) for acquiring detection signals, which detection signals depend on the magnetization in the field of view (28), which magnetization is influenced by the change in the position in space of the first and second sub-zone (52, 54),
   - control means (150) for controlling said signal generator units (110, 120, 130) to generate and provide control currents to the respective field coils to generate appropriate magnetic fields for moving the catheter (190) through the object (180) in a direction instructed by movement commands and for localizing the catheter (190) within the object (180), and
   - processing means (154) for processing said detection signals acquired when appropriate magnetic fields are applied for localizing the catheter (190) within the object (180) and for determining the position of the magnetic element (194) of the catheter (190) within the object (180) from the processed detection signals.

2. An apparatus (100) as claimed in claim 1, wherein said control means (150) is adapted for controlling said signal generator units (110, 120, 130) to generate and provide control currents to the respective field coils to alternately generate appropriate magnetic fields for moving the catheter (190) through the object (180) in a direction instructed by movement commands and for localizing the catheter (190) within the object (180).

3. An apparatus (100) as claimed in claim 1, wherein said control means (150) is adapted for converting manual or predetermined movement commands into control signals for controlling said signal generator units (110, 120, 130).

4. An apparatus (100) as claimed in claim 1, further comprising a catheter (190) movement means (160) for providing a forward and backward movement of the catheter (190).

5. An apparatus (100) as claimed in claim 4, wherein said control means (150) is adapted for controlling said catheter (190) movement means (160).

6. An apparatus (100) as claimed in claim 5, wherein said control means (150) is adapted for controlling said catheter (190) movement means such that during localization of said catheter (190) no forward or backward movement is applied on the catheter (190), in particular such that said catheter (190) is kept in position.

7. An apparatus (100) as claimed in claim 1, further comprising focus means comprising a focus field signal generator unit (120) and focus field coils (126a, 126b, 126c) for changing the position in space of the field of view (28) by means of a magnetic focus field,

8. An apparatus (100) as claimed in claim 1 or 7, wherein said control means (150) is adapted for controlling said focus field signal generator unit (120) and/or said drive field generator unit (130) to generate and provide control currents to the focus field coils (126a, 126b, 126c) and/or said drive field coils (136a, 136b, 136c) to generate substantially homogeneous magnetic fields for moving the catheter (190) through the object (180) in a direction instructed by movement commands.

9. An apparatus (100) as claimed in claim 8, wherein said control means (150) is adapted for controlling said selection field signal generator unit (110) to generate and provide no control current to the selection field coils (116) while magnetic fields are generated by said focus field coils (126a, 126b, 126c) and/or said drive field coils (136a, 136b, 136c) for moving the catheter (190) through the object (180) in a direction instructed by movement commands.

10. A method for controlling an apparatus as defined in any one of the preceding claims to control the movement of a catheter (190) through an object (180) and localize the catheter (190) within the object (180), said catheter (190) comprising a magnetic element (194) at or near its tip (192), which method comprises the steps of controlling said apparatus to carry out the steps of:

- generating a magnetic selection field (50) having a pattern in space of its magnetic field strength such that a first sub-zone (52) having a low magnetic field strength and a second sub-zone (54) having a higher magnetic field strength are formed in a field of view (28),
- changing the position in space of the two sub-zones (52, 54) in the field of view (28) by means of a magnetic drive field so that the magnetization of magnetic material in the field of view changes locally,
- acquiring detection signals, which detection signals depend on the magnetization in the field of view (28), which magnetization is influenced by the change in the position in space of the first and second sub-zone (52, 54),
- controlling the generation of appropriate magnetic fields for moving the catheter (190) through the object (180) in a direction instructed by movement commands and for localizing the catheter (190) within the object (180), and
- processing said detection signals acquired when appropriate magnetic fields are applied for localizing the catheter (190) within the object (180) and for determining the position of the magnetic element (194) of the catheter (190) within the object (180) from the processed detection signals.

11. Computer program comprising program code means for causing a computer to control an apparatus as claimed in claim 1 to carry out the steps of the method as claimed in claim 10 when said computer program is carried out on the computer.

**Patentansprüche**

1. Gerät (100) zum Steuern der Bewegung eines Katheters (190) durch ein Objekt (180) und zum Lokalisieren des Katheters (190) innerhalb des Objekts (180), wobei der genannte Katheter (190) ein Magnetelement (194) an oder nahe seiner Spitze (192) umfasst, wobei das Gerät Folgendes umfasst:

- Auswahlmittel umfassend eine Auswahlfeldsignalgeneratoreinheit (110) und Auswahlfeldelemente (116), insbesondere Auswahlfeldmagneten oder -spulen, zum Erzeugen eines magnetischen Auswahlfelds (50) mit einem derartigen räumlichen Muster seiner Magnetfeldstärke, dass eine erste Teilzone (52) mit einer niedrigen Magnetfeldstärke und eine zweite Teilzone (54) mit einer höheren Magnetfeldstärke in einem Sichtfeld (28) gebildet werden,
- Ansteuerungsmittel umfassend eine Ansteuerungsfeldsignalgeneratoreinheit (130) und Ansteuerungsfeldspulen (136a, 136b, 136c) zum Ändern der räumlichen Position der beiden Teilzonen (52, 54) in dem Sichtfeld (28) mithilfe eines magnetischen Ansteuerungsfelds, so dass sich die Magnetisierung des magnetischen Materials in dem Sichtfeld lokal ändert,
- Empfangsmittel umfassend mindestens eine Signalempfangseinheit (140) und mindestens eine Empfangsspule (148) zum Erfassen von Detektionssignalen, wobei die Detektionssignale von der Magnetisierung im Sichtfeld (28) abhängen, wobei die Magnetisierung durch die Änderung der räumlichen Position der ersten und der zweiten Teilzone (52, 54) beeinflusst wird,
- Steuermittel (150) zum Steuern der genannten Signalgeneratoreinheiten (110, 120, 130), um Steuerströme zu erzeugen und den jeweiligen Feldspulen bereitzustellen, um geeignete Magnetfelder zum Bewegen des Katheters (190) durch das Objekt (180) in einer durch Bewegungsbefehle angewiesenen Richtung und zum Lokalisieren des Katheters (190) innerhalb des Objekts (180) zu erzeugen, und

- Verarbeitungsmittel (154) zum Verarbeiten der genannten Detektionssignale, die erfasst werden, wenn geeignete Magnetfelder angelegt werden, um den Katheter (190) innerhalb des Objekts (180) zu lokalisieren und um die Position des Magnetelements (194) des Katheters (190) innerhalb des Objekts (180) anhand der verarbeiteten Detektionssignale zu ermitteln.

2. Gerät (100) nach Anspruch 1,
wobei die genannten Steuermittel (150) dafür ausgelegt sind, die genannten Signalgeneratoreinheiten (110, 120, 130) zu steuern, um Steuerströme zu erzeugen und den jeweiligen Feldspulen bereitzustellen, um abwechselnd geeignete Magnetfelder zum Bewegen des Katheters (190) durch das Objekt (180) in einer durch Bewegungsbefehle angewiesenen Richtung und zum Lokalisieren des Katheters (190) innerhalb des Objekts (180) zu erzeugen.

3. Gerät (100) nach Anspruch 1,
wobei die genannten Steuermittel (150) dafür ausgelegt sind, manuelle oder vorbestimmte Bewegungsbefehle in Steuersignale zum Steuern der genannten Signalgeneratoreinheiten (110, 120, 130) umzuwandeln.

4. Gerät (100) nach Anspruch 1,
weiterhin umfassend ein Bewegungsmittel (160) für den Katheter (190), um eine Vorwärts- und Rückwärtsbewegung des Katheters (190) bereitzustellen.

5. Gerät (100) nach Anspruch 4,
wobei die genannten Steuermittel (150) dafür ausgelegt sind, die genannten Steuermittel (160) für den Katheter (190) zu steuern.

6. Gerät (100) nach Anspruch 5,
wobei die genannten Steuermittel (150) dafür ausgelegt sind, die genannten Bewegungsmittel für den Katheter (190) derartig zu steuern, dass während der Lokalisierung des genannten Katheters (190) keine Vorwärts- oder Rückwärtsbewegung auf den Katheter (190) angewendet wird, insbesondere derartig, dass der genannten Katheter (190) in seiner Position gehalten wird.

7. Gerät (100) nach Anspruch 1,
weiterhin umfassend Fokusmittel umfassend eine Fokusfeldsignalgeneratoreinheit (120) und Fokusfeldspulen (126a, 126b, 126c) zum Ändern der räumlichen Position des Sichtfelds (28) mithilfe eines magnetischen Fokusfelds.

8. Gerät (100) nach Anspruch 1 oder 7,
wobei die genannten Steuermittel (150) dafür ausgelegt sind, die genannte Fokusfeldsignalgeneratoreinheit (120) und/oder die genannte Ansteuerungsfeldsignalgeneratoreinheit (130) zu steuern, um Steuerströme zu erzeugen und den Fokusfeldspulen (126a, 126b, 126c) und/oder den genannten Ansteuerungsfeldspulen (136a, 136b, 136c) bereitzustellen, um im Wesentlichen homogene Magnetfelder zum Bewegen des Katheters (190) durch das Objekt (180) in einer durch Bewegungsbefehle angewiesenen Richtung zu erzeugen.

9. Gerät (100) nach Anspruch 8,
wobei die genannten Steuermittel (150) dafür ausgelegt sind, die genannte Auswahlfeldsignalgeneratoreinheit (110) zu steuern, um keinen Steuerstrom zu erzeugen und den Auswahlfeldspulen (116) bereitzustellen, während durch die genannten Fokusfeldspulen (126a, 126b, 126c) und/oder die genannten Ansteuerungsfeldspulen (136a, 136b, 136c) Magnetfelder erzeugt werden, um den Katheter (190) in einer durch Bewegungsbefehle angewiesenen Richtung durch das Objekt (180) zu bewegen.

10. Verfahren zum Steuern eines Gerät, wie es in einem der vorhergehenden Ansprüche definiert ist, um die Bewegung eines Katheters (190) durch ein Objekt (180) zu steuern und den Katheter (190) innerhalb des Objekts (180) zu lokalisieren, wobei der genannte Katheter (190) ein Magnetelement (194) an oder nahe seiner Spitze (192) umfasst, wobei das Verfahren die Schritte des Steuerns des genannten Geräts umfasst, damit es die folgenden Schritte ausführt:

- Erzeugen eines magnetischen Auswahlfelds (50) mit einem derartigen räumlichen Muster seiner Magnetfeldstärke, dass eine erste Teilzone (52) mit einer niedrigen Magnetfeldstärke und eine zweite Teilzone (54) mit einer höheren Magnetfeldstärke in einem Sichtfeld (28) gebildet werden,

- Ändern der räumlichen Position der beiden Teilzonen (52, 54) in dem Sichtfeld (28) mithilfe eines magnetischen Ansteuerungsfelds, so dass sich die Magnetisierung des magnetischen Materials in dem Sichtfeld lokal ändert,

- Erfassen von Detektionssignalen, wobei die Detektionssignale von der Magnetisierung im Sichtfeld (28) abhängen, wobei die Magnetisierung durch die Änderung der räumlichen Position der ersten und der zweiten Teilzone (52, 54) beeinflusst wird,

- Steuern der Erzeugung von geeigneten Magnetfeldern zum Bewegen des Katheters (190) durch das Objekt (180) in einer durch Bewegungsbefehle angewiesenen Richtung und zum Lokalisieren des Katheters (190) innerhalb des Objekts (180), und

- Verarbeiten der genannten Detektionssignale, die erfasst werden, wenn geeignete Magnetfelder angelegt werden, um den Katheter (190) innerhalb des Objekts (180) zu lokalisieren und um die Position des Magnetelements (194) des Katheters (190) innerhalb des Objekts (180) anhand der verarbeiteten Detektionssignale zu ermitteln.

**11.** Computerprogramm umfassend Programmcodemittel zum Veranlassen eines Computers, ein Gerät nach Anspruch 1 zu steuern, damit es die Schritte des Verfahrens nach Anspruch 10 durchführt, wenn das genannte Computerprogramm auf dem Computer ausgeführt wird.

## Revendications

**1.** Appareil (100) pour la commande du mouvement d'un cathéter (190) à travers un objet (180) et la localisation du cathéter (190) dans l'objet (180), ledit cathéter (190) comprenant un élément magnétique (194) au niveau de sa pointe (192) ou à proximité de celle-ci, ledit appareil comprenant :

- des moyens de sélection comprenant une unité génératrice de signaux de champ de sélection (110) et des éléments de champ de sélection (116), en particulier des aimants ou des bobines de champ de sélection, pour générer un champ de sélection magnétique (50) ayant un motif dans l'espace de son intensité de champ magnétique de sorte qu'une première sous-zone (52) ayant une intensité de champ magnétique faible et une seconde sous-zone (54) ayant une intensité de champ magnétique plus élevée soient formées dans un champ de vision (28),
- des moyens d'entraînement comprenant une unité génératrice de champ d'entraînement (130) et des bobines de champ d'entraînement (136a, 136b, 136c) pour modifier la position dans l'espace des deux sous-zones (52, 54) dans le champ de vision (28) au moyen d'un champ d'entraînement magnétique de sorte que l'aimantation du matériau magnétique dans le champ de vision change localement,
- des moyens de réception comprenant au moins une unité réceptrice de signaux (140) et au moins une bobine réceptrice (148) pour acquérir des signaux de détection, lesquels signaux de détection dépendent de l'aimantation dans le champ de vision (28), ladite aimantation étant influencée par le changement de position dans l'espace de la première et la seconde sous-zone (52, 54),

- des moyens de commande (150) pour commander lesdites unités génératrices de signaux (110, 120, 130) afin de générer et fournir des courants de commande aux bobines de champ respectives pour générer des champs magnétiques appropriés pour déplacer le cathéter (190) à travers l'objet (180) dans une direction instruite par des instructions de mouvement et pour localiser le cathéter (190) dans l'objet (180), et
- des moyens de traitement (154) pour traiter lesdits signaux de détection acquis lorsque des champs magnétiques appropriés sont appliqués pour localiser le cathéter (190) dans l'objet (180) et déterminer la position de l'élément magnétique (194) du cathéter (190) dans l'objet (180) à partir des signaux de détection traités.

**2.** Appareil (100) selon la revendication (1), dans lequel lesdits moyens de commande (150) sont à même de commander lesdites unités génératrices de signaux (110, 120, 130) pour générer et fournir des courants de commande aux bobines de champ respectives pour générer en alternance des champs magnétiques appropriés afin de déplacer le cathéter (190) à travers l'objet (180) dans une direction instruite par des instructions de mouvement et pour localiser le cathéter (190) dans l'objet (180).

**3.** Appareil (100) selon la revendication 1, dans lequel lesdits moyens de commande (150) sont à même de convertir des instructions de mouvement manuelles ou prédéterminées en signaux de commande afin de commander lesdites unités génératrices de signaux (110, 120, 130).

**4.** Appareil (100) selon la revendication 1, comprenant en outre des moyens (160) de déplacement du cathéter (190) pour assurer un mouvement en avant et en arrière du cathéter (190).

**5.** Appareil (100) selon la revendication 4, dans lequel lesdits moyens de commande (150) sont à même de commander lesdits moyens (160) de mouvement du cathéter (190).

**6.** Appareil (100) selon la revendication 5, dans lequel lesdits moyens de commande (150) sont à même de commander lesdits moyens de mouvement du cathéter (190) de sorte que, au cours de la localisation dudit cathéter (190), aucun mouvement en avant ou en arrière ne soit appliqué au cathéter (190), en particulier de sorte que ledit cathéter (190) soit maintenu en position.

**7.** Appareil (100) selon la revendication 1, comprenant en outre des moyens de focalisation comprenant une unité génératrice de signaux de champ de focalisation (120) et des bobines de champ de focalisation (126a, 126b, 126c) pour changer la position dans l'espace du champ de vision (28) au moyen d'un champ de focalisation magnétique.

8. Appareil (100) selon la revendication 1 ou 7, dans lequel lesdits moyens de commande (150) sont à même de commander ladite unité génératrice de signaux de champ de focalisation (120) et/ou ladite unité génératrice de champ d'entraînement (130) pour générer et fournir des courants de commande aux bobines de champ de focalisation (126a, 126b, 126c) et/ou auxdites bobines de champ d'entraînement (136a, 136b, 136c) pour générer des champs magnétiques sensiblement homogènes pour déplacer le cathéter (190) à travers l'objet (180) dans une direction instruite par des instructions de mouvement.

9. Appareil (100) selon la revendication 8, dans lequel lesdits moyens de commande (150) sont à même de commander ladite unité génératrice de signaux de champ de sélection (110) pour ne pas générer et ne pas fournir de courant de commande aux bobines de champ de sélection (116) tandis que des champs magnétiques sont générés par lesdites bobines de champ de focalisation (126a, 126b, 126c) et/ou lesdites bobines de champ d'entraînement (136a, 136b, 136c) pour déplacer le cathéter (190) à travers l'objet (180) dans une direction instruite par des instructions de mouvement.

10. Procédé de commande d'un appareil selon l'une quelconque des revendications précédentes, pour commander le mouvement d'un cathéter (190) à travers un objet (180) et localiser le cathéter (190) dans l'objet (180), ledit cathéter (190) comprenant un élément magnétique (194) au niveau de sa pointe (192) ou à proximité de celle-ci, ledit procédé comprenant les étapes de commande dudit appareil pour effectuer les étapes consistant à :

- générer un champ de sélection magnétique (50) ayant un motif dans l'espace de son intensité de champ magnétique de sorte qu'une première sous-zone (52) ayant une intensité de champ magnétique faible et une seconde sous-zone (54) ayant une intensité de champ magnétique plus élevée soient formées dans un champ de vision (28),
- changer la position dans l'espace des deux sous-zones (52, 54) dans le champ de vision (28) au moyen d'un champ d'entraînement magnétique de sorte que l'aimantation du matériau magnétique dans le champ de vision change localement,
- acquérir des signaux de détection, lesquels signaux de détection dépendent de l'aimantation dans le champ de vision (28), l'aimantation étant influencée par le changement de position dans l'espace de la première et de la seconde sous-zone (52, 54),
- commander la génération de champs magnétiques appropriés pour déplacer le cathéter (190) à travers l'objet (180) dans une direction instruite par des instructions de mouvement et pour localiser le cathéter (190) dans l'objet (180), et
- traiter lesdits signaux de détection acquis lorsque des champs magnétiques appropriés sont appliqués pour localiser le cathéter (190) dans l'objet (180) et pour déterminer la position de l'élément magnétique (194) du cathéter (190) dans l'objet (180) à partir des signaux de détection traités.

11. Programme informatique comprenant des moyens de codage de programme pour amener un ordinateur à commander un appareil selon la revendication 1 pour effectuer les étapes du procédé selon la revendication 10 lorsque ledit programme informatique est effectué sur l'ordinateur.

FIG. 1

**FIG. 2**

FIG. 3

FIG. 4

FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 10151778 A1
- US 20030125752 A1
- EP 1304542 A
- WO 2004091386 A
- WO 2004091390 A
- WO 2004091394 A
- WO 2004091395 A
- WO 2004091396 A
- WO 2004091397 A
- WO 2004091398 A
- WO 2004091408 A

**Non-patent literature cited in the description**

- **GLEICH, B. ; WEIZENECKER, J.** Tomographic imaging using the nonlinear response of magnetic particles. *nature,* 2005, vol. 435, 1214-1217